# EUROPEAN PATENT APPLICATION

(11) **EP 4 464 324 A1**
(43) Date of publication of application: **20.11.2024**
(21) Application number: 24176726.8
(22) Date of filing: 17.05.2024
(51) Int. Cl.: A61K 31/728, A61K 33/00, A61P 15/00, A61K 9/00

(54) **ASSOCIATION OF GASEOUS MOLECULAR OXYGEN AND HYALURONIC ACID FOR USE IN THE TREATMENT OF INFERTILITY IN SUBJECTS WITH VAGINAL DYSBIOSIS AND/OR A SERIES OF ASSISTED FERTILISATION FAILURES**

(30) Priority: 18.05.2023 IT 202300010119
(71) Applicant: Caress Flow S.r.l., 40050 Argelato (BO) (IT)
(72) Inventor: Montanari, Federico, 40050 Argelato BO (IT); Garoia, Flavio, 40050 Argelato BO (IT); Venturi, Flavio, 40050 Argelato BO (IT)
(74) Representative: Perani & Partners S.p.A.

(57) **Abstract**

The present invention relates to an association comprising molecular oxygen and hyaluronic acid for use in the treatment of infertility of female subjects presenting vaginal dysbiosis and/or a series of assisted fertilization failures.

## Description

### FIELD OF THE INVENTION

The present patent application relates to an association comprising gaseous molecular oxygen and hyaluronic acid for use in the treatment of infertility of female subjects with vaginal dysbiosis and/or a series of assisted fertilization failures.

### STATE OF THE ART

The human microbiota consists of trillions of microorganisms, a much higher number than that of the cells of the human body, with which the microbiota coexists without damaging it. It consists of bacteria, fungi, viruses, protozoa, and represents a super-organism weighing about one and a half kilograms and protecting us from numerous diseases. Each person has a distinct and highly variable composition of gut microorganisms, although there is a fixed core of microorganisms common to all individuals. The composition of microorganisms is called the microbiota, while all of the genes in the microbiota are called the microbiome. There are about 150 times the number of genes in the gut microbiome than genes in the human body.

The human microbiota practically resides throughout our body: for the most part (more than 70%) it is found in the gastrointestinal tract, but in reality it is also present in other body districts, such as: skin, hair, oral cavity, genital organs, different tracts of the airways (nostrils, external auditory canal). The microbiota populates the digestive tract and other organs of the human body; protects the intestinal mucosa and other mucous membranes; regulates natural immune defences; and limits the growth of pathogenic bacteria. The microbiota allows the production of some of the essential vitamins for our body, such as folic acid, vitamin K, vitamins in the B group, contributes to the regulation of intestinal peristalsis and the proper functioning of various organs, including those responsible for reproduction.

We host many more microbes than we are capable of growing in the laboratory: one gram of intestinal contents contains about 1 billion bacteria. There is a continuous dialogue between our cells and the microbial community we host, a relationship which is very effective and capable of strongly influencing our state of health. It is known that the microbiota plays an active role in important vital functions, such as digestion and maturation of the intestinal mucosa, intervenes in immune mechanisms to recognize and counter potential threats and to avoid colonization by pathogenic infectious agents, plays an important role in diseases such as obesity or diabetes or other metabolic diseases, and is even capable of modulating our mood.

Our microbiota is formed at birth, and then it changes in relation to our diet, our lifestyle, the environment in which we live and where we work, also based on the diseases which affect us and the pharmacological therapies we take.

The alteration of the microbiota, both in *number* and *composition,* can result in disorders, even disease states.

It is known that the woman's microbiota changes during pregnancy and/or in the first weeks after conception: the number of bacteria present in the intestines increases, but above all the percentages of the different bacterial families vary. All in relation to the change in metabolism necessary for the development and birth of the unborn child.

### Vaginal microbiota

The vaginal microbiota is characterized by five types of microbial communities (CST, *Community State Types*) in asymptomatic women. Four of them (CST-I, II, III, V) are dominated by *Lactobacillus* species, while CST-IV is heterogeneous and polymicrobial, characterized by a lower level of *Lactobacillus* and a higher level of anaerobic bacteria, including *Gardnerella, Atopobium, Mobiluncus, Prevotella, Streptococcus, Mycoplasma* and *Ureaplasma.*

Currently over 140 species of *Lactobacillus* have been identified, but the only species which normally dominate the vaginal microbiota are: *L. crispatus* (CST-I), *L. gasseri* (CST-II), *L. iners* (CST-III) and *L. jensenii* (CST-V). They are considered milestones of vaginal health, as most are capable of producing lactic acid, hydrogen peroxide H₂O₂ and/or bacteriocins, maintaining an acidic environment and preventing the growth of pathogens, for example by rejecting the adhesion thereof to the epithelium. They are capable of regulating the immune and inflammatory response, increasing the vagina's resistance to disease. Therefore, lactobacilli dominance is generally considered a hallmark of a healthy vagina.

The vaginal microbiota is therefore an important factor involved in the protection against pathogenic bacteria, fungi and viruses and, generally, in the absence of symptoms or infections, is associated with a correct functioning of the reproductive system. The vaginal microbiota is essential for the health of the female genital system, for the maintenance of reproductive function and for the proper course of pregnancy.

It is hypothesized that the vaginal microbiota is formed primarily by the advent of microbes from the rectum.

*Lactobacillus* are present in healthy women of reproductive age and act as probiotics capable of inhibiting the growth of pathogenic bacteria, viruses and fungi, for example through a lowering of the vaginal pH, mediated by the production of lactic acid. The activity of the lactobacilli helps to maintain the balance of the vaginal microbiota; this role is fundamental for the health of the vagina, because, as mentioned above, the lactobacilli produce about 80% lactic acid - in addition to proteins, carbohydrates, glycoproteins, lipothetic acids and divalent cations -, and because they form a "natural barrier" from pathogens and chemical stress.

The number of lactobacilli species, as well as the composition of the vaginal microbiota, are related to a woman's age, menstrual cycle, sexual activity and smoking habits.

It is known, for example, that oestrogens support the proliferation of the vaginal epithelium and formation of intraepithelial glycogen, while progesterone supports the cytolysis of epithelial cells, which release glycogen which is readily metabolised by lactobacilli into glucose, maltose and lactic acid.

The condition of vaginal dysbiosis (RV or unhealthy dysmicrobial or microbiota) is described by three main changes in the vaginal environment:
- a change in the composition of the vaginal microbiota, from *Lactobacillus spp.* to optional anaerobes;
- the production of amino compounds by the new bacterial microbiota;
- an increase in vaginal pH to more than 4.5.

Other factors involved in the balance of the vaginal microbiota are: dietary factors (nutritional deficiencies, diets abundant in carbohydrates and/or fats, obesity); lifestyle (smoking, stress, use of aggressive detergents etc.).

### Infertility

The World Health Organization (WHO) considers infertility a disease and defines it as the absence of conception after 12/24 months of regular unprotected targeted sexual intercourse.

Infertility in Italy affects about 15% of couples while, in the world, about 10-12%. This disease can concern the man, the woman or both (couple infertility). However, it may also occur that there is an impossibility for that particular union between individuals to conceive life.

Female infertility, responsible for 35-40% of couple infertility, can depend on several causes, such as: hormonal alterations, tubal alterations, uterine diseases, patient age, systemic or genetic diseases. Below are several factors capable of inducing infertility in women:
*Hormonal factor*: polycystic ovary syndrome (PCOS), thyroid disorders or a reduced ovarian reserve can cause changes in the menstrual cycle up to anovulation (absence of ovulation).

*Tubal factor*: abnormalities in the morphology and function of the fallopian tubes. In fact, the tubes are the organs which allow the female and male gametes to meet; to do this, the patency of the tubes, the integrity and functionality of the tubal mucosa and its muscular apparatus are necessary to ensure the contractile activity thereof. The most frequent causes of tubal damage are pelvic infections (PID); the most frequent and dangerous microorganism is *Chlamydia,* a sexually transmitted bacterium with a marked tubal tropism. Other causes can be the results of abdominal surgery, endometriosis, congenital forms of malformations often associated with also malformations of the uterus.

*Uterine factor*: structural alterations of the uterus which interfere with procreative capacity. Congenital (uterine septum, bicornuate uterus or uterine subseptus) or acquired malformations (polyps, myomas, synechiae) can hinder conception or be responsible for more or less early abortions, as they cause alterations of the uterine cavity.

*Cervical factor*: morphological alterations (cervical agenesis, cervical septum, adhesions) or functional alterations (density of cervical mucus, inflammation) in the uterine cervix can prevent or hinder the ascent of sperm in the uterus and towards the tubes. There are also plasma cells in the cervical tissue which produce specific antibodies which, in certain conditions, can reduce fertility, interfering with sperm migration and viability.

*Systemic diseases, nutritional factors, lifestyle habits:* hypothalamic-pituitary causes (Kallmann's syndrome, hyperprolactinaemia, hypopituitarism, Cushing's syndrome), diabetes mellitus, thyroid disorders, diseases of the adrenal glands, liver or kidney diseases. Exposure to toxic substances, such as agricultural pesticides, solvents present in paints, exposure to heavy metals (mercury, arsenic, lead, cadmium, etc.), radiation and high temperature can also be dangerous for fertility; even the intake of dangerous drugs for fertility or narcotic substances can have a negative effect on fertility.

*Age factor*: the reproductive capacity of the couple undergoes a decline with advancing age; the female age is of particular importance, as the woman is born with a determined follicular heritage which gradually depletes. Female fertility undergoes a first significant decline, albeit gradual, already around the age of 32, and a second more rapid decline after the age of 37, to be close to zero in the years before menopause. This feature, on the one hand, limits the time useful for procreation, and on the other hand, explains the increased incidence of miscarriages in the first trimester with increasing age, because the quality of the oocytes also decreases.

The diagnosis of infertility is based on hormonal dosages and instrumental investigations.

The hormonal dosages most frequently requested by the gynaecologist are: FSH, LH, estradiol and prolactin to be performed between the 2nd and 3rd day of the menstrual cycle and progesterone on the 21st day; TSH, fT4 and AMH (anti-Mullerian hormone), which can be performed independently of the cycle.

The instrumental investigations most frequently requested are: pelvic ultrasound (to check the ovarian follicle count, the occurrence of ovulation and the presence of any lesions of the uterus and adnexal); hysterosalpingography; hysterosonography/salpingography; hysteroscopy.

As mentioned, the vagina is an important and complex ecosystem dominated by *Lactobacillus,* but also containing a small number of fungi and parasites, and the balanced microbial communities are vital for women's health.

Differences between the vaginal microbiota of pregnant women with respect to non-pregnant women lead to the hypothesis that, physiologically, gestation causes changes in the microbial composition of the vaginal environment. In this sense, the study of the vaginal microbiome is being studied in relation to female infertility.

The microbial balance can be altered and lead to various *infectious* diseases, characterized by overgrowth of anaerobic bacteria (agents of bacterial vaginitis and atrophic vaginitis, BV and AV), *Candida albicans* (agent of vulvovaginal candidiasis, VVC), as well as infections of *Trichomonas vaginalis* (agent of trichomonial vaginitis), *Neisseria gonorrhoeae* (agent of gonorrhoea), *Mycoplasma genitalium* (agent of cervicitis), *Chlamydia trachomatis* (agent of pelvic inflammatory disease, PID), and various viruses including human papillomavirus (HPV, agent of cervical cancer), *herpes simplex virus-2* (HSV-2, agent of genital ulcers), and human immunodeficiency virus (HIV, agent of acquired immunodeficiency syndrome, AIDS).

Furthermore, some *non-infectious* diseases, for example miscarriages (with BV microbiome), preterm birth (with BV microbiome), infertility (with BV microbiome), polycystic ovary syndrome (Polycystic Ovary Syndrome or PCOS, with reduction of *Lactobacillus crispatus* and increase of mycoplasmas and *Prevotella*), show a connection with the condition of bacterial vaginosis, which therefore represents a serious threat to the reproductive health of women.

Numerous evidence has shown that BV (bacterial vaginosis) could increase the risk of preterm birth and infertility. Furthermore, it is known that women who cannot conceive due to idiopathic infertility are often characterized by an altered presence of the cervico-vaginal microbiome, whose composition is similar to that found in the case of BV, which sees an increase in colonization by anaerobic bacteria such as *Atopobium, Prevotella, Veillonella, Ureaplasma* and *Escherichia.* In this sense, the presence of the bacterium *Atopobium vaginae* in the vaginal microbiome has been found as one of the main factors contributing to the failure of *in vitro* fertilization, as well as embryo transfer procedures, in women with asymptomatic bacterial infections.

In a picture of infertility, some species of lactobacilli are less represented, against considerable colonization by others; for example, *L. iners* is more represented both in a picture of (not idiopathic) infertility, and in that of idiopathic infertility. Furthermore, *L. gasseri* has been identified in greater quantities in embryo culture media, follicular fluids and seminal fluids in subjects with idiopathic infertility with respect to subjects with non-idiopathic infertility (Lactobacillus iners and gasseri, Prevotella bivia and HPV Belong to the Microbiological Signature Negatively Affecting Human Reproduction, G. Campisciano et al, Microorganisms 2021, 9, 39).

Furthermore, the protocols for IVF (*In Vitro Fertilization*) provide a study opportunity to investigate vaginal microbiome composition fluctuations in a well-defined hormonal context. Indeed, high hormone levels induced by controlled hyperstimulation of the ovary during IVF have been correlated with changes in the vaginal microbiome and increased susceptibility of the woman to vaginal infections.

The association between an altered presence of vaginal microorganisms and a lower pregnancy rate in patients undergoing IVF has recently been demonstrated by Haahr T. and colleagues (Skafte-Holm A, Humaidan P, Bernabeu A, Lledo B, Jensen JS, Haahr T. The Association between Vaginal Dysbiosis and Reproductive Outcomes in Sub-Fertile Women Undergoing IVF-Treatment: A Systematic PRISMA Review and Meta-Analysis. Pathogens. 2021 Mar 4;10(3):295. doi: 10.3390/pathogens10030295. PMID: 33806442; PMCID: PMC8001118).

Many studies have also reported that about 20-30% of women suffer, at least once, from a deficiency of *Lactobacillus* in their vaginal microflora. Some types of lactobacilli are more associated with poor reproductive outcomes (e.g., *L. iners*), and sexually transmitted diseases, while some *Lactobacillus* species, particularly *Lactobacillus crispatus,* are more related to vaginal health.

Knowing the *composition* of the vaginal microbiome could be an important element both to identify the causes of female infertility in cases hitherto classified as idiopathic, and to develop personalized therapeutic interventions which aim to restore the balance of the vaginal environment and promote conception and the continuation of pregnancy.

Currently, the most conventional treatment strategy for disorders of the vaginal microbiota, such as vaginal dysbiosis in connection with infertility, is the use of antibiotics in association with probiotics.

### Problems of the background art

However, antibiotics (such as metronidazole, clotrimazole, azithromycin, etc.), show a good therapeutic effect, but are accompanied by various adverse effects and relapses, as well as plausible development of antibiotic resistance.

The results of treatment with probiotics or yeasts are generally mixed, which may be due to the fact that these pathological conditions are usually caused by *multiple* microbes, rather than just one. The actual absence or reduced incidence of side effects related to taking probiotics or yeasts is still under debate.

There is, therefore, a need to identify an association of at least two active substances capable of treating infertility in female subjects presenting vaginal dysbiosis and/or a series of assisted fertilisation failures, in a more efficient, safe, low-cost, non-invasive manner, and which can be easily administered, possibly also in a domestic context or by a non-medical operator.

It should further be noted that the female subjects are infertile and, in this sense, would like to become pregnant; for this reason, it is necessary to pay special attention to the drugs used for the aforesaid purposes.

Finally, the need is felt to rebalance the number and/or composition of lactobacilli of the vaginal microbiome in female subjects with vaginal and non-fertile dysbiosis, with possible series of medically assisted fertilization failures.

### SUMMARY OF THE INVENTION

The Applicant has developed an association (or combination) comprising molecular oxygen and hyaluronic acid for use in the treatment of infertility of female subjects with vaginal dysbiosis and/or a series of assisted fertilization failures.

A further object of the invention is a kit comprising
- the association for the aforesaid use,
- at least one generator of molecular oxygen or in gaseous form,
- the formulation comprising hyaluronic acid,
- means for delivering the molecular oxygen and means for applying the hyaluronic acid in a topical vaginal form, preferably at least one cannula.

### Advantages of the invention

The association of the invention is advantageous because:
- it effectively treats the infertility of female subjects presenting vaginal dysbiosis and/or a series of assisted fertilization failures;
- it is not invasive, nor are any particular side effects observed;
- it is inexpensive and easily applicable, even by a little experienced or inexperienced user;
- it is capable of rebalancing the number and/or composition of lactobacilli in the vaginal microbiome in non-fertile female subjects, especially lactobacilli capable of promoting reproduction (see Example 1);
- the molecular oxygen and hyaluronic acid comprised in the association of the invention support, promote and/or assist each other's action (mutual promotion of the biological activity of the two active ingredients).

### DETAILED DESCRIPTION OF THE INVENTION

The Applicant is aware of the use of the association of molecular oxygen and hyaluronic acid for the treatment of vaginal disorders or infections, such as vulvovaginal atrophy, vaginal dryness and candidiasis.

Association is intended as the combination of molecular or gaseous oxygen with hyaluronic acid, the latter preferably comprised in a formulation.

Vulvovaginal atrophy is a disease mainly linked to a lower oestrogen concentration and the onset of menopause. Candidiasis is instead an infectious disease, induced by the *Candida* fungus. The known use of the association comprising molecular oxygen and hyaluronic acid for vulvovaginal atrophy and recurrent candidiasis seems to resolve the symptoms associated with the two diseases, and is capable of lowering the concentration of *Candida* for the latter.

On the other hand, although the picture of one or the other disease could be associated with a condition of vaginal dysbiosis, the Applicant considers that, from the known uses of the association, the resolution of the condition of vaginal dysbiosis cannot be derived, in terms of physiological rebalancing of the number and/or composition of lactobacilli in the vaginal environment. Therefore, the Applicant ignores the use of the association of molecular oxygen and hyaluronic acid in the treatment of female subjects presenting vaginal dysbiosis and/or a series of assisted fertilization failures.

Oxygen concentrations in the human vagina are known to be low (about 10%).

Lactobacilli, mainly constituting the vaginal microbiome, are strictly *anaerobic* bacteria (they hardly live or grow in the presence of oxygen). They can also be found in oxygen-rich niches; however, in these cases, it seems that, in order to reduce the concentration of oxygen - otherwise harmful - several lactobacilli, but not all, are capable of producing H₂O₂. *In vitro* experiments have demonstrated that isolates of *L. crispatus, L. jensenii* and *L. gasseri* were superior to *L. iners* in the number of H₂O₂ producing isolates.
*L. iners* is part of the normal vaginal microbiota and is in fact one of the main vaginal species; however, its role in vaginal health is ambiguous. *L. iners* has an unusually smaller genome with respect to other lactobacilli of the vagina, which is indicative of a parasitic and symbiotic lifestyle. It is also known that *L. iners* produces a specific Cholesterol-Dependent Cytolysin (CDC), "inerolycin", which is a pore-forming toxin, similar to the action of the vaginolysin produced by *Gardnerella sp.* It can therefore be hypothesized that the presence of *L. iners,* as the main colonizer, offers a favourable environment for the survival *of Gardnerellla sp.,* destabilizing the vaginal microbiota, especially due to the limited amount of lactic acid produced. More and more evidence suggests that *L. iners* provides less protection against infectious species, and is more prone to the acquisition of bacterial vaginosis than other lactobacilli.

### Association comprising molecular oxygen and hyaluronic acid

The association (or combination) of the invention comprises or consists of molecular oxygen and hyaluronic acid for use in the treatment of infertility of female subjects with vaginal dysbiosis and/or a series of assisted fertilization failures.

Preferably, the association (or combination) comprises or consists of
molecular oxygen or oxygen in gaseous form and
a formulation comprising, in turn:
   - at least one active ingredient consisting of hyaluronic acid,
   - suitable excipients and/or diluents,
and is for use in the treatment of infertility of female subjects with vaginal dysbiosis and/or a series of assisted fertilization failures.

### Molecular oxygen in gaseous form

The association of the invention comprises active molecular (or gaseous) oxygen.

Preferably, the molecular oxygen (or gaseous oxygen) has a degree of purity ranging between ≥ 90% (v/v), preferably between 90% and 99%, preferably between 90% and 96% (v/v), preferably between 92% and 95% (v/v), preferably equal to 95% (v/v).

The molecular oxygen comprised in the association can be obtained using methodologies known to the person skilled in the art; preferably, the molecular oxygen is obtained thanks to an oxygen concentrator, which sucks the external air and concentrates it in the oxygen fraction. The maximum delivery pressure by means of the oxygen concentrator is preferably ranging between 50 and 150 kPa, preferably it is 100 kPa.

The dosage of molecular oxygen to be administered is preferably ranging between 5 and 130 L, preferably ranging between 5 and 120 L, preferably between 10 and 130 L, preferably ranging between 10 and 120 L, preferably between 20 and 120 L, preferably between 10 and 90 L, preferably between 10 and 70 L.

According to a preferred form of the invention, the molecular oxygen is delivered at a flow ranging preferably between 0.1 L/min and 7 L/min, preferably between 0.5 and 7 L/min, preferably between 1 and 6 L/min, preferably between 1.5 and 4.5 L/min, preferably equal to 3 L/min.

### Hyaluronic acid and formulation comprising hyaluronic acid

The association comprises a second active ingredient consisting of hyaluronic acid.

Preferably, the hyaluronic acid is comprised in a formulation, the latter not comprising the molecular oxygen or oxygen in gaseous form.

Preferably, hyaluronic acid is the only active ingredient comprised in the formulation, the latter comprising suitable excipients and/or diluents.

Preferably, hyaluronic acid is comprised in a formulation in solid, liquid or semi-solid form.

### Ist embodiment: hyaluronic acid comprised in the solid formulation

Preferably, the hyaluronic acid is comprised in a solid formulation.

The solid formulation, preferably in the form of a vaginal ovule, comprises or consists of:
- the active ingredient consisting of hyaluronic acid, and
- suitable excipients and/or diluents.

The solid formulation is preferably for topical vaginal use. The solid formulation is preferably in the form of a vaginal ovule.

Preferably, suitable excipients and/or diluents for the preparation of vaginal ovules are known to the person skilled in the art. For example, water-soluble excipients are often used, which melt at body temperature, but dissolve slowly in vaginal secretions; therefore, they are able to gradually release, in a continuous and controlled manner, the contained active ingredients. Examples of hydrophilic excipients are: glycerinated gelatin; diols (e.g., methylpropanediol, 1,2-Hexanediol and 1,2-Octanediol); polyethylene glycols (PEG) or mixtures of the foregoing.

For the preparation of vaginal ovules, lipophilic excipients, for example of the type used for the preparation of suppositories, may also be preferably employed. These lipophilic excipients also melt at body temperature, but they release the active ingredient quickly and completely, without remaining in contact with the vaginal mucosa for a time from which systemic effects can be potentially derived. Examples of lipophilic excipients are: synthetic and/or semi-synthetic glycerides and/or of vegetable origin, for example mono-, di- and triglycerides esters of fatty acids, preferably with a number of carbon atoms ranging between 8 and 10, preferably saturated fatty acids, with a predominance of the triester fraction; cocoa butter and/or its derivatives.

For the preparation of vaginal ovules, excipients selected from the group consisting of: gelatin, glycerinated gelatin, diols, PEGs and/or their derivatives, synthetic and/or semi-synthetic glycerides and/or of vegetable origin, cocoa butter and/or its derivatives, moisturisers, humectants, dyes, preservatives, pH modifiers, diluents, flavourings, water, polymers that increase mucoadhesion (acrylic acid derivatives, or cellulose derivatives), rheological modifiers, thickeners, stabilizers, gelling agents, and mixtures of the foregoing may preferably be included.

Preferably, the hyaluronic acid comprised in the formulation is in an amount ranging between 0.05% and 1% by weight, preferably between 0.07% and 1% by weight, preferably between 0.1% and 0.5% by weight, preferably between 0.2% and 0.3% by weight, preferably equal to 0.2% by weight, on the total weight of the formulation.

In the solid formulation, the hyaluronic acid preferably has a molecular weight ranging between 100 kDa and 250 kDa, preferably between 180 kDa and 230 kDa.

Preferably, the hyaluronic acid comprised in the formulation is a derivative of hyaluronic acid, preferably an ester derivative of hyaluronic acid, preferably it is an ester of hyaluronic acid with benzyl alcohol; preferably, 50% of the carboxylic groups of hyaluronic acid are esterified with the corresponding hydroxyl groups of benzyl alcohol.

### 2nd embodiment: hyaluronic acid comprised in the liquid formulation

Preferably, the hyaluronic acid is comprised in a liquid formulation.

The liquid formulation, preferably in the form of an aqueous solution, comprises or consists of:
- hyaluronic acid active ingredient, and
- suitable excipients and/or diluents.

The liquid formulation is preferably for topical use, preferably it is applied to the external area of the female genitalia, or it is applied to the inside of the vagina (topical, intravaginal application) with a suitable medium, preferably a medium suitable for the nebulization of hyaluronic acid, for example a cannula.

Preferably, suitable excipients and/or diluents for the preparation of vaginal solutions are known to the person skilled in the art. For example, excipients/diluents selected from the group consisting of: solvents (water), humectants, conditioners, chelants, antistatic agents, emulsifiers, surfactants, preservatives, buffer systems, pH modifiers, flavourings, deodorants, rheological agents, antioxidants may preferably be included.

Preferably, the excipients and/or diluents are selected from the group consisting of (INCI names): aqua, disodium EDTA, PVP (or Polyvinylpyrrolidone), phenoxyethanol, benzoic acid, dehydroacetic acid, ethylhexylglycerin, sodium hydroxide, lactic acid, perfume, benzyl alcohol, methyl benzoate, Butylhydroxytoluene (BHT), PEG-40 hydrogenated castor oil, and mixtures of the foregoing.

Preferably, the hyaluronic acid comprised in the formulation is in an amount ranging between 0.05% and 1% by weight, preferably between 0.07% and 1% by weight, preferably between 0.1% and 0.5% by weight, preferably between 0.2% and 0.3% by weight, preferably equal to 0.2% by weight, on the total weight of the formulation.

Preferably, the dosage of hyaluronic acid is ranging between 10 mg and 120 mg per dosage unit.

In the liquid formulation, the hyaluronic acid preferably has a molecular weight ranging between 40 kDa and 1400 kDa.

According to a preferred embodiment, the liquid formulation comprises: hyaluronic acid at a molecular weight ranging between 40 kDa and 60 kDa, and hyaluronic acid at a molecular weight ranging between 1000 kDa and 1400 kDa.

The amount of water comprised in the liquid formulation ranges between 95% and 98% by weight, preferably between 96% and 98% by weight, on the total weight of the liquid formulation.

The liquid formulation is preferably in the form of a bottle, preferably disposable; preferably, each bottle has a volume ranging between 10 and 20 ml, preferably 15 ml.

### 3rd embodiment: hyaluronic acid comprised in the semi-solid formulation

A semi-solid formulation is preferably in the form of a gel, emulgel, foam, mousse.

Preferably, the hyaluronic acid is comprised in a semi-solid formulation.

The semi-solid formulation, preferably in the form of a gel or mousse, comprises or consists of:
- hyaluronic acid active ingredient, and
- suitable excipients and/or diluents.

The semi-solid formulation is preferably for topical use, preferably it is applied to the outer area of the female genitalia.

Such a semi-solid formulation may contain excipients and/or diluents, selected from the group consisting of: chelators, surfactants, emulsifiers, humectants, solubilisers, rheological agents, perfumes, preservatives, buffer systems, pH modifiers, and mixtures of the foregoing.

Preferably, the hyaluronic acid comprised in the formulation is in an amount ranging between 0.05% and 1% by weight, preferably between 0.07% and 1% by weight, preferably between 0.1% and 0.5% by weight, preferably between 0.2% and 0.3% by weight, preferably equal to 0.2% by weight, on the total weight of the formulation.

The Applicant considers that the hyaluronic acid comprised in the formulation exerts its combined effect with that of oxygen in gaseous form vaginally *regardless* of the embodiment of the formulation comprising hyaluronic acid.

Preferably, the association of the invention and the formulation comprising hyaluronic acid - *regardless* of the embodiment of the formulation - do not contain: antibiotics and/or probiotics and/or bacteria and/or yeasts and/or hyaluronidase inhibitors.

### Use of the association of the invention

The association (or combination) of the invention comprises or consists of molecular oxygen and hyaluronic acid for use in the treatment of infertility of female subjects with vaginal dysbiosis and/or a series of assisted fertilization failures. In other words, the female subjects are infertile and have a condition of vaginal dysbiosis VD, whereby the VD can occur in the presence or not of a series of assisted fertilization failures.

Infertility is intended as a disease which consists of no conception after 12/24 months of regular unprotected targeted sexual intercourse.

Subjects with (or presenting) vaginal dysbiosis is intended as female subjects presenting a condition of vaginal dysbiosis, i.e., a condition of the vaginal environment characterized by an imbalance in terms of the number and/or composition of the lactobacilli physiologically constituting the vaginal microbiome.

Under physiological conditions (or microbiota profile dominated by lactobacilli), the vaginal concentration of *Lactobacillus spp,* preferably *L. crispatus, L. gasseri, L. jensenii* and *L. iners,* is ≥ 90% with respect to the total microbiota.

Under conditions of vaginal dysbiosis, the vaginal concentration of *Lactobacillus spp,* preferably *L. crispatus, L. gasseri, L. jensenii* and *L. iners,* is <90% with respect to the total microbiota.

According to a preferred form, the association of molecular oxygen and hyaluronic acid is for use in the treatment of infertility, preferably idiopathic infertility, of female subjects with vaginal dysbiosis and/or a series of assisted fertilization failures.

Bacterial vaginosis (BV) is intended as a particular form of vaginal dysbiosis.

It is generally known that the alteration of the vaginal microbiota is related to various gynaecological diseases, in particular BV, which is characterized by the alteration of the vaginal microbiome from the dominance of lactobacilli to that of anaerobic and optional bacteria, for example optional anaerobic bacteria selected from: *Gardnerella, Atopobium, Prevotella, Megasphaera, Leptotrichia, Sneathia,* and combinations of the foregoing.

BV is defined as an alteration of the vaginal microbiota, which results in a heterogeneous and dysbiotic vaginal environment, with reduced concentrations of *Lactobacillus spp.* and an increased presence of bacteria typical of BV, such as *Gardnerella spp.* Furthermore, the BV may involve the presence of a polymicrobial biofilm which is strongly adherent to the vaginal epithelium. BV is associated with infertility, failure of assisted fertilization techniques, miscarriage, preterm birth. The presence of H₂O₂-producing lactobacilli is negatively associated with the condition of BV.

In microbiota profiles dominated by BV (or BVB) bacteria, a concentration > 10% of anaerobic bacteria, other than *Lactobacillus spp.,* is observed, preferably chosen from the species: *Gardnerella, Atopobium, Prevotella, Megasphaera, Leptotrichia, Sneathia,* and combinations of the foregoing.

"Series of assisted fertilization failures" is intended as a number ≥ 2, preferably ≥ 2 and ≤ 6, preferably ≥ 2 and ≤ 5, preferably ≥ 3 and ≤ 4, of unsuccessful attempts at assisted fertilization techniques or medically assisted procreation for a female subject.

The assisted fertilization or medically assisted procreation (MAP) techniques are those commonly known in the medical field. For example, assisted fertilization techniques are intended as:
- level I techniques: Intrauterine Insemination (IUI), or
- level II techniques such as: *In Vitro* Fertilization (IVF) or *In Vitro* Embryo-Transfer Fertilization (IVFET), or IntraCytoplasmic Sperm Injection (ICSI).

Idiopathic (or idiopathic-type) infertility is intended as a type of infertility for which the causes are not known or cannot be demonstrated with diagnostic technology. In particular, idiopathic infertility is defined as the condition for which it is not possible to conceive after 12 months of attempts, despite regular ovulation and sperm parameters and in the absence of other physical impediments.

Female subjects of childbearing age is intended as female subjects of reproductive age; preferably, such subjects have a chronological age ranging from 18 to 40 years, preferably ranging from 18 to 37 years, preferably ranging from 20 to 37 years.

Preferably, the association of the invention is for topical vaginal use, preferably it is applied inside the vaginal cavity.

Preferably, the association of the invention is for use as an adjuvant in the therapies for the treatment of infertility, preferably idiopathic infertility, of female subjects having vaginal dysbiosis, preferably bacterial vaginosis, and/or a series of assisted fertilization failures.

For the aforesaid purposes, adjuvant means the association of the invention which, in combination with at least one medical therapy known for the aforesaid purposes (for example, at least one chosen among: a hormonal and/or antibiotic and/or probiotic-based therapy and/or a MAP technique), completes and/or increases the effectiveness of action thereof.

### Kit comprising the association of the invention

A further object of the invention is a kit, preferably a kit for use in infertility in subjects presenting vaginal dysbiosis and/or a series of assisted fertilization failures, comprising:
- the association of the invention comprising molecular oxygen and hyaluronic acid, or the association comprising oxygen in gaseous form and the formulation comprising the active ingredient hyaluronic acid and suitable excipients and/or diluents,
- at least one generator of molecular oxygen (or in gaseous form),
- the formulation comprising hyaluronic acid, preferably in solid or liquid form, preferably in the liquid form of a solution,
- means for applying and/or delivering molecular oxygen topically vaginally, preferably at least one cannula, preferably at least one disposable cannula, preferably at least one disposable cannula with at least one gas outlet hole,
- optionally, connection means, preferably connection means between the at least one generator of oxygen in gaseous form and the means for delivering the molecular oxygen topically vaginally, for example a cannula,
- optionally, means for regulating the flow of molecular oxygen, preferably a flow meter,
- optionally, instructions for the kit and/or the relative use.

It should be noted that the connection means can preferably be pipes in a material suitable for containing a flow of molecular oxygen or in gaseous form, preferably plastic pipes.

### Therapeutic protocol

Preferably, the method according to the invention comprises the following steps:
- delivering the molecular oxygen in gaseous form topically vaginally or step (a);
- after the aforesaid step (a), delivering the molecular oxygen in gaseous form topically vaginally and, at the same time, topically applying the hyaluronic acid or step (b), preferably topically applying a formulation comprising hyaluronic acid, the formulation preferably being in solid (vaginal ovule), liquid (aqueous solution) or semi-solid form.

The protocol is preferably performed fortnightly, preferably for a total of five weeks.

### Step (a)

Preferably, step (a) has a duration preferably ranging between 3 and 15 minutes, preferably ranging between 5 and 10 minutes, preferably equal to 10 minutes.

Preferably, step (a) above provides for the delivery of the active ingredient oxygen in gaseous form topically vaginally through the use of a suitable delivery means, such as for example a cannula, preferably a cannula comprising at least one gas outlet hole, preferably a disposable cannula.

Preferably, the means suitable for delivering/applying oxygen in gaseous form is inserted into the vaginal cavity, preferably starting from the external access of the vaginal opening, gradually entering the interior, until contact with the "tench snout" of the uterine cervix.

In anatomy, "tench snout" refers to the intravaginal segment of the cervix. Cone-shaped, with the trunk apex facing downwards, the tench snout typically measures 8-12 mm in length. The apex has an orifice (lower orifice of the cervix or external orifice of the uterus) that enters the uterine cavity.

### Step (b)

Preferably, step (b) envisages delivering the molecular oxygen in gaseous form topically vaginally and, at the same time, topically applying the hyaluronic acid or step (b), preferably topically applying a formulation comprising hyaluronic acid, the formulation preferably being in solid (vaginal ovule), liquid (aqueous solution) or semi, form.

Step (b) has a duration preferably ranging between 2 and 10 minutes, preferably ranging between 5 and 10 minutes, preferably equal to 5 minutes.

In a preferred form, the hyaluronic acid is previously dissolved in distilled water, so as to form a 0.2% (w/v) solution (liquid formulation or solution, according to the 2nd embodiment of the invention).

Preferably, the application of the liquid formulation is topical, preferably intravaginal.

Specifically, the treatment preferably envisages the introduction of the hyaluronic acid solution, injecting it into the special valve of the vaginal cannula (nebulization of the aqueous solution), at the same time as the delivery of gaseous oxygen.

### EXAMPLES

Below, the Applicant sets out examples for illustrative but not limiting purposes.

### Example 1 - In vivo evaluation test of the association of the invention

Five oxygen therapy treatments were performed fortnightly (5 cases), for a total of five weeks. The items were evaluated at the beginning and end of the treatment.

The treatment is performed using a device known with the trade name of Exea X2, which transforms air into 95% pure gaseous oxygen. The Exea X2 device consists of:
*Compressor:* a compressed air generator with the function of sucking air from the external environment, filtering and compressing it.

*Machine body:* provided with zeolite molecular sieves. It exploits the principle of different absorption of gas molecules by different surfaces, allowing O₂ to pass through and retaining other gases in the air, such as nitrogen, argon, helium and hydrogen. The machine body transforms the outside air into 95% pure oxygen.

*Dispenser:* consisting of a vaginal cannula, connected to the machine body. The cannula is provided with outlet holes for the delivery of oxygen and hyaluronic acid, which is inserted by means of a special injection valve, located at the top of the cannula.

The treatment protocol was as follows:
i) delivery of oxygen with vaginal cannula, for a duration of 10 minutes;
ii) subsequently, delivery of vaginal oxygen in combination with the application of hyaluronic acid, for a duration of 5 minutes.

Five female subjects of childbearing age with a condition of vaginal dysbiosis were treated.

The measurements, vaginal swab and vaginal microbiota collection, were performed before the first treatment (T0) and two weeks after the fifth treatment (T5).

The results show a repopulation of all the major species of lactobacilli post-treatment; specifically, a reduction of *L. iners* is observed, concomitant with an increase, in particular, of *L. crispatus.*

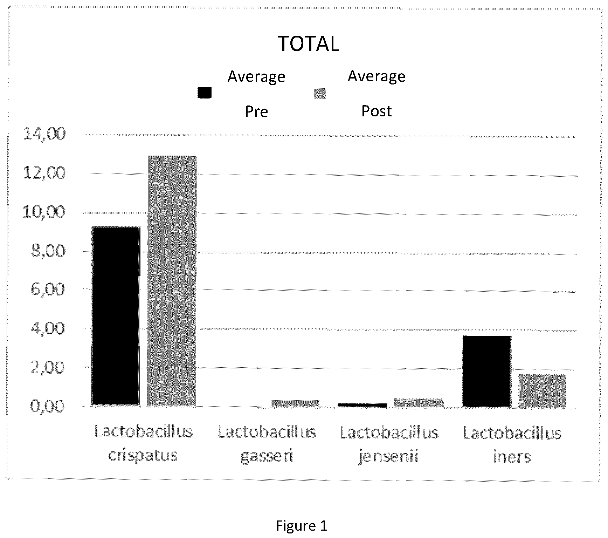

## Claims

1. Association comprising molecular oxygen and hyaluronic acid for use in the treatment of infertility of female subjects presenting vaginal dysbiosis and/or a series of assisted fertilization failures.

2. Association for the use according to claim 1, wherein the infertility is of idiopathic type.

3. Association for the use according to claim 1 or 2, wherein the series of assisted fertilization failures is equal to a number ≥ 2, preferably ≥ 2 and ≤ 6.

4. Association for the use according to any one of claims from 1 to 3, wherein the association is for vaginal topical use.

5. Association for the use according to any one of claims from 1 to 4, wherein the molecular oxygen has a degree of purity ≥ 90% by volume (v/v).

6. Association for the use according to any one of claims from 1 to 5, wherein the molecular oxygen dosage is comprised between 5 and 130 litres.

7. Association for the use according to any one of claims from 1 to 6, wherein the hyaluronic acid is comprised in a formulation in solid, liquid or semi-solid form, preferably the formulation is in liquid form.

8. Association for the use according to any one of claims from 1 to 7, wherein the hyaluronic acid is comprised in a formulation in an amount comprised between 0.05% and 1% by weight on the total weight of the formulation.

9. Association for the use according to claim 7 or 8, wherein the hyaluronic acid is the only active ingredient comprised in the formulation.

10. Kit comprising
- the association for the use according to any one of claims from 1 to 9,
- at least one generator of molecular oxygen or in gaseous form,
- the formulation comprising hyaluronic acid according to any one of claims from 7 to 9,
- means for delivering the molecular oxygen and means for applying the hyaluronic acid in a topical vaginal form, preferably at least one cannula.
